# EUROPEAN PATENT APPLICATION

(11) **EP 4 152 005 A1**
(43) Date of publication of application: **22.03.2023**
(21) Application number: 22195929.9
(22) Date of filing: 15.09.2022
(51) Int. Cl.: G01N 33/68

(54) **CARDIOVASCULAR SCREENING USING CONJUNCTIVAL MICROCIRCULATORY PARAMETERS AND BLOOD BIOMARKERS**

(30) Priority: 15.09.2021 GB 202113149
(71) Applicant: Randox Laboratories Ltd., Crumlin Antrim BT29 4QY (GB)
(72) Inventor: Ruddock, Mark, Crumlin, BT29 4QY (GB); Fitzgerald, Peter, Crumlin, BT29 4QY (GB)

(57) **Abstract**

Methods and systems for supporting cardiovascular disease diagnosis are described which incorporate the proteins adiponectin and NT-proBNP in conjunction with conjunctival haemodynamic parametric measurement. The methods and systems can benefit from the use of mobile devices to improve their portability and ease of use.

## Description

### Background to the Invention

Cardiovascular disease (CVD) is a major cause of morbidity and mortality in developed countries. It accounts for 17.9 million deaths globally, with up to 80% of these deaths due to myocardial infarction (MI) or stroke. Coronary artery disease (CAD) is the most common type of CVD and manifests as atherosclerosis in the coronary arteries which leads to angina and myocardial infarction (MI). CAD is the leading cause of death in developed countries. CVD not only carries a significant morbidity and mortality burden but has major economic implications. The World Heart Foundation estimated the total cost of CVD was US$863 billion in 2010, with a predicted increase to approximately US$1,044 billion by 2030. The total expenditure on CVD in the EU is €210 billion annually with 53% due to direct health care expenditures, productivity losses cost 26%, and informal care of patient costs 21%. Atherosclerosis is the key pathophysiological process that underlies CAD. This process develops over a prolonged period and is influenced by a variety of medical and behavioural risk factors. Atherosclerotic plaques in general do not produce symptoms until the affected vessel has significant luminal narrowing and the resulting ischaemic cascade can be silent in its early stages. Prior to atherosclerotic plaque rupture and resultant MI, patients may be asymptomatic. Many predictive approaches, screening and diagnostic methods for atherosclerotic diseases have evolved. These include utilization of independent traditional and biochemical risk factors, general CVD risk scores, and assessment of cardiac microvasculature. Current European Society of Cardiology (ESC) clinical guidelines recommend the systematic assessment of cardiovascular (CV) risk in men greater than 40 years-old and in women greater than 50 years-old or post-menopausal with no CV risk factors. ESC assessment of risk for asymptomatic individuals utilises the Systematic Coronary Risk Estimation (SCORE) assessment tool, whilst the National Institute for Clinical Excellence (NICE) advocate the use of QRISK. Both tools take into consideration conventional vascular risk factors to estimate long-term CV risk. The results of these estimates of risk are then used to inform GPs' decisions with regards to primary preventative therapies and overall long-term risk. The risk score obtained may be modified by the presence of pre-existing medical conditions that were not taken into consideration during the initial risk calculation e.g. chronic kidney disease or diabetes mellitus; or with the detection of preclinical asymptomatic vascular damage using imaging modalities e.g. computed tomography (CT) coronary calcium score, carotid ultrasound or ankle brachial pressure index. All pre-existing screening methods are limited either by the exposure of the patient to ionizing radiation or by the need for expensive imaging modalities requiring operator expertise to perform and interpret, such as carotid ultrasound. Due to the global and regional health and socioeconomic implications of CAD, there is a need to identify novel factors for screening asymptomatic individuals for both early detection and occurrence of CAD. Previously heart-type fatty acid-binding protein (H-FABP), for example, has been shown to be an early blood diagnostic biomarker of MI. H-FABP is detected in blood within 30 minutes of an ischaemic event. Measurement of H-FABP with troponin is a reliable diagnostic tool for the early diagnosis of MI and a valuable rule out test at presentation to the emergency department. Measurement of biomarkers in addition to cholesterol may provide further information to identify individuals at risk of CAD in asymptomatic individuals. Microcirculatory studies (blood flow in arterioles, capillaries and venules) have demonstrated the association of inflammation with atherosclerosis. In addition, it has been shown that endothelial dysfunction in the microcirculation is an early manifestation and marker of vascular disease. Microcirculatory changes have been observed in patients with hypertension, diabetes, sickle cell disease and sepsis. The capillary networks of the human eye are sufficiently accessible to allow examination of the microcirculation at the anterior (ocular adnexum) and posterior (retina) aspect. Changes to the retinal vessel diameters show evidence of diseases that are conventional risk factors for CAD e.g. diabetes and hypertension, which could play a pathophysiological role in CVD risk stratification and prediction. Similarly, blood flow within the conjunctival vasculature can be visualised and hence assessment of blood microcirculatory (haemodynamic) parameters (e.g. velocity (V), flow (Q), wall shear rate (WSR)) can also be measured non-invasively. Previous studies have identified conjunctival microvascular changes in diseases such as sickle cell, hypertension and diabetes mellitus and more recently it was suggested that conjunctival microvascular parameter measurements were different in MI patients compared to control (Brennan, P. F. et al. 2021. Assessment of the conjunctival microcirculation for patients presenting with acute myocardial infarction compared to healthy controls. Scientific Reports, 11, 1-9).

### Summary of the Invention

There remains a need to develop improved CVD screening diagnostics that could be used to economically, rapidly and effectively to support the identification of patients at risk of CAD and who are experiencing or have recently experienced MI. Improved methods of CAD risk assessment and MI are described which combine measurements of conjunctival microcirculatory parameters (CMP) and blood biomarkers, the former further supplemented by the use of readily available, cost-effective and portable measurement devices. A first aspect of the invention provides a method of aiding the diagnosis of CAD or MI, comprising using measurements of conjunctival microcirculatory parameters in conjunction with measurements of NT-proBNP and adiponectin in an ex *vivo* sample of a patient. The patient can be apparently healthy, suspected of experiencing or having experienced MI or subject to CAD. The measurements are compared to reference sample values and an assessment is made as to whether CAD or MI is present. A further aspect of the invention describes the use of measurements of the two ex *vivo* blood biomarkers and microcirculatory parameter(s) to implement the diagnosis of CAD risk or MI, its effectiveness enhanced as the microcirculatory parameter data that is used in the methods of the invention is derived by incorporating personal mobile devices that do not require the input of qualified clinicians/specialists or expensive, complex and bulky medical devices, hence are readily placeable in GP surgeries and mobile testing units.
**Figure 1** ROC curve for combination of Vs, NT-proBNP and adiponectin
**Figure 2** Schematic of a system capable of implementing the described methods

### Detailed Description of the Invention

In a first aspect of the invention is described a method of coronary artery disease detection comprising measuring the concentration of each of NT-proBNP and adiponectin in an *in vitro* sample of the patient and by using the acquired measurement data with patient conjunctival haemodynamic data the presence or absence of cardiovascular disease can be detected. By obtaining or taking a sample from the patient i.e. an *in vitro* sample, and measuring the concentration of each of the proteins NT-proBNP and adiponectin in the obtained *in vitro* sample, and using these measurements in conjunction with physiological measurement data relating to conjunctival haemodynamic parameters of the patient, especially that relating to flow velocity, it is possible to identify patients with cardiovascular disease, and especially those patients who are suffering from coronary artery disease or who are suffering from or who have recently suffered a myocardial infarction. Cardiovascular disease refers to conditions affecting the heart and blood vessels and includes coronary heart disease or atherosclerosis (fatty-acid build-up in the arteries), angina, heart failure, heart attack or myocardial infarction, ischaemic stroke, transient ischaemic attack (TIA), peripheral arterial disease which represents blockages in limbs such as the legs, and aortic disease. Haemodynamic parameters include diameter (d), axial velocity (Va), cross-sectional velocity (Vs), wall shear rate/stress (WSR/WSS) and blood volume flow (Q); resistance to blood flow in a single vessel is described by Poiseuille's Equation (see Klabunde, R.E., Cardiovascular Physiology Concepts, Lippincott Williams & Wilkins second edition, 2012). In a preferred embodiment the method of the invention makes use of data derived from cross-sectional and/or axial velocity measurements. It has been found that the levels or concentration in an *in vitro* sample of a patient with MI or CAD compared to a control sample of the biomarker NT-proBNP is greater whereas that of adiponectin is lower (see Table 3); the axial and cross-sectional velocity measurement data of CVD patients have also been found to be lower (see Table 2). The methods described support the diagnosis of CVD, which with clinician support and/or other biomarker (e.g. H-FABP) and/or physiological analysis (e.g. ECG), can lead to or support a diagnosis of MI or CVD. The aforementioned measurement data derived from either i. NT-proBNP, adiponectin and Vs or ii. NT-proBNP, adiponectin and Va can be combined in a computer algorithm that can incorporate a statistical method to facilitate analysis and produce an output which indicates whether CVD is present or absent. The algorithm can incorporate biomarker and CMP semi-quantitative data which can take the form of a simple cut-off value measurement for each which can provide a 'yes'/'no' output for the three measurements i.e. yes, the biomarker and CMP level in the patient is greater than the cut-off; no, the biomarker level and CMP in the patient is less than the cut-off (or a mixture of yes/no). Examples of suitable mathematical/statistical methods for use in a computer algorithm include binary logistic regression, multinominal logistic regression, multilayer perceptron neural network (MLP), artificial neural networks, support vector machines, N Bayes classification and random forest classifiers. The accuracy of statistical methods used in accordance with the present invention can be also be described by their receiver operating characteristics (ROC). The ROC curve addresses both the sensitivity, the number of true positives, and the specificity, the number of true negatives, of the test. Therefore, sensitivity and specificity values for a given combination of biomarkers and CMP are an indication of the accuracy of the assay. For example, if a biomarker and CMP combination has sensitivity and specificity values of 80%, out of 100 patients which have CAD, 80 will be correctly identified from the determination of the presence of the particular combination of biomarkers as positive for CAD, while out of 100 patients who have not suffered a MI 80 will accurately test negative for the disease. Sensitivity and specificity values are defined by the cut-off value assigned to the biomarker/CMP - depending upon the sensitivity or specificity required in an assay i.e. whether a 'rule in' or 'rule out' assay is required, the cut-off value assigned will vary. If two or more biomarkers/CMP are to be used in the diagnostic method a suitable mathematical model, such as a logistic regression equation, can be derived. The logistic regression equation can include other variables such as age and gender of patient. The ROC curve can be used to assess the accuracy of the logistic regression model. The logistic regression equation can be used as an independent algorithm or as part of an algorithm to aid clinical decision making. Although a logistic regression equation is a common mathematical/statistical procedure used in such cases and is preferred in the context of the present invention, other mathematical/statistical procedures can also be used. Thus in a further aspect there is a method of aiding cardiovascular disease detection comprising measuring the level of NT-proBNP and adiponectin in an *in vitro* sample of the patient and by using the acquired measurement data with patient conjunctival haemodynamic data and placing both sets of data into a computer algorithm the presence or absence of coronary artery disease in the patient is assessed using the computed output. The skilled person will be aware of numerous suitable methods for developing mathematical/statistical algorithms to process the measurement data, and all of these are within the scope of the present invention; the following binary logistic regression (Equation 1) is exemplary and supports a diagnostic sensitivity and specificity of 93% and 91.5%, respectively (see Table 4).

*Equation 1: Patient score= -15.462* + *11.516 logVs* + *-3.22 log[adiponectin]* + *2.423 log[NT-proBNP]* (Patient score=0.736, Vs =cross sectional velocity, [adiponectin]= concentration of adiponectin in µg/ml, [NT=proBNP] is the concentration of NT-proBNP in ng/L; a patient whose score exceeds 0.736 is positive for CAD).

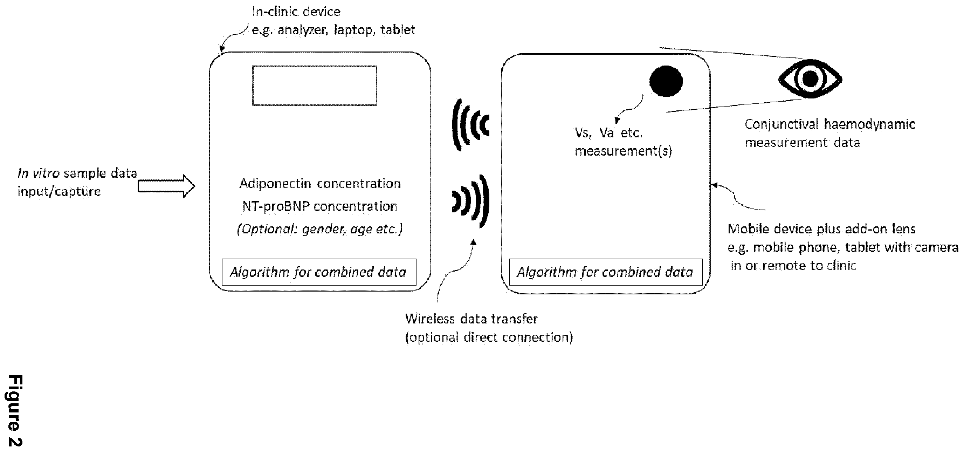

The output may be a numerical value, which indicate presence or absence of CAD, can be an explanation of the findings, or a different representation such as a tick or cross or a colour code, or combination of the different ouputs; the haemodynamic data is preferably derived from the axial or cross-sectional velocity measurement. The device used for conjunctival haemodynamic data acquisition is small, portable and affordable enabling the methods of the invention to be applicable outside of mainstream hospital settings such as in GP surgeries, pop-up (easily and rapidly constructed) and mobile medical units, and at a patient's residence. Furthermore, it enables the acquisition of conjunctival haemodynamic measurements without the need of a trained technician. In a further aspect of the invention is a method of aiding cardiovascular disease detection comprising measuring the level of NT-proBNP and adiponectin in an *in vitro* sample of the patient in conjunction with conjunctival haemodynamic measurement data derived from conjunctival haemodynamic parameters such as Va and Vs, in which the conjunctival haemodynamic measurement(s) is/are derived from a system that comprises a mobile device incorporating photographic means and one or more computer programs for processing, analysing and producing an output relating to the captured data; the mobile device can also incorporate measurement data derived from the *in vitro* patient sample and other input and/or stored data all of which can feed into the computer program(s). It is preferable that the computer program(s) further incorporates an algorithm based on binary logistic regression; equation 1 described one such algorithm. The mobile device can be a laptop computer, tablet, or a mobile phone, each having photographic means. In a preferred embodiment the mobile device is a mobile phone and is preferably used with an attachable lens to increase magnification. A mobile phone with an attachable lens that allows magnification to view blood vessels at the front of the eye and allow CMP measurements is described in Brennan et al., Scientific Reports 11, article number: 7660 (2021). A further aspect of the invention is a system that can be used to support the diagnosis of CAD or MI. The system comprises a mobile device with photographic means which incorporates a computer program for processing data relating to an haemodynamic conjunctival parameter measurement and communication means to a device incorporating a computer program incorporating an algorithm for processing data relating to a haemodynamic conjunctival parameter, adiponectin and NT-proBNP. The mobile device with photographic means and the device comprising a computer program incorporating the algorithm can be co-located e.g. both are in the same room in a hospital or GP surgery, or can be at discrete locations e.g. different locations within a hospital, different addresses in same town, different towns etc. In a preferred embodiment the mobile device with photographic means and the device comprising a computer program incorporating the algorithm are discretely located. For example, the mobile device with photographic means can be in a mobile unit that is despatched to GP surgery or patients home in order to obtain the CMP measurements, whereas the device comprising the computer program incorporating the algorithm is located at a hospital. Following acquisition of a patient biological sample this can be analysed at the hospital and the resultant data stored on the device; following CMP data acquisition this can be transmitted to the hospital to the device comprising the computer program incorporating the algorithm and stored biomarker data and the combined data can be processed. This has the advantage of improving patient data security as a supporting diagnosis is only possible at the hospital upon receipt of the CMP data. Alternatively, the acquired biomarker data results can be transmitted (from the hospital) to a patient mobile device with photographic means, the mobile device also incorporating the computer program with algorithm; when combined with separately acquired CMP data, information relating to CVD diagnosis can be provided securely and directly to the patient (see Figure 2).

The terms "subject", "individual" and "patient" may be used interchangeably herein and refer to a mammal including a non-primate (e.g. a cow, pig, horse, dog, cat, rat and mouse) and a primate (e.g. a monkey and human). Preferably the individual or patient is a human. As used herein, the term 'biomarker' refers to a molecule present in a biological sample obtained from a patient, the concentration of which in said sample may be indicative of a pathological state. It is well understood in the art that biomarker normal or background concentrations may exhibit slight variation due to, for example, age, gender or ethnic/geographical genotypes as well as well-known experimental variation relating to the experimenter and the devices used. As a result, the constant and coefficient values of any mathematical/statistical formulae used to support CVD diagnosis may also slightly vary. The ex *vivo* biological sample used in the described methods is any suitable sample type but preferably a blood, serum or plasma sample. As used herein, the term *'in vitro sample'* (or 'ex *vivo sample')* has its usual meaning in the art and refers to a biological sample that is not in an individual's body. Analysis of the blood sample can be by way of several analytical methodologies such as mass spectrometry linked to a pre-separation step such as chromatography. The preferred methodology is based on immuno-detection. Immuno-detection technology is also readily incorporated into transportable or handheld devices for use outside of the clinical environment e.g. at the patient's residence and which do not require a trained technician to effect. A quantitative immunoassay such as a Western blot or ELISA can also be used to detect the amount of protein. Another method of analysis comprises using a multi-analyte biochip which enables several proteins to be detected and quantified simultaneously. A biochip is a planar substrate that may be, for example, mineral or polymer based, but is preferably ceramic. Probes are adsorbed on or chemically attached to the surface of the biochip. The probes can be any biomarker-specific probe or binding ligand. As used herein, the term 'specific' means that the probe binds only to one of the biomarkers of the invention, with negligible binding to other biomarkers of the invention or to other analytes in the biological sample being analysed. This ensures that the integrity of the diagnostic assay and its result using the biomarkers of the invention is not compromised by additional binding events. When identifying the various biomarkers of the invention it will be apparent to the skilled person that as well as identifying the full-length protein, the identification of a fragment or several fragments of a protein is possible, provided this allows accurate identification of the protein. Similarly, although a preferred probe of the invention is a polyclonal or monoclonal antibody, other probes such as aptamers, molecular imprinted polymers, phages, short chain antibody fragments and other antibody-based probes may be used. If, a solid-state device is used in the methods of the present invention, the Biochip Array Technology system (BAT) (available from Randox Laboratories Limited) is preferable. Also, the Evidence Evolution and Evidence Investigator apparatus (available from Randox Laboratories) may be used to determine the levels of biomarkers in the sample using BAT. The term "NT-proBNP" as used herein refers to the protein number P16860 in the Uniprot Knowledgebase database. The term "adiponectin" as used herein refers the protein number Q15848 in the Uniprot Knowledgebase database. The described method of diagnosing or diagnosing the risk of CVD comprises measuring the level of adiponectin, NT-proBNP in a sample taken/obtained from a patient and comparing the measured level to a corresponding reference value. Use of the words/terms 'level', 'measured level' and 'reference value' etc. in the singular form of noun in the various aspects of the invention that refer to 'one or more of..", also signifies the plural noun forms 'levels', 'measured levels' and 'reference values' etc. The reference value or control value is the level or concentration of the biomarkers NT-proBNP, adiponectin and the CMP measured in samples taken from (i.e. an ex *vivo* or *in vitro* sample) the healthy patient prior to CAD onset or diagnosis or from a healthy cohort (group). In all of the described methods patient diagnosis can be supported through a combination of data derived from further biomarker measurements other than NT-proBNP and adiponectin, patient physiological measurements and data, and clinician examination and input.

### Methods

*Study population:* a cohort of inpatients with severe cardiovascular phenotype (CAD) after an acute MI was compared with a healthy gender-matched patient cohort. Participants <18 years were excluded from the study. All study participants were recruited between January 2018 and November 2019. Participants were eligible for inclusion in the 'healthy' cohort if they had no previous history of CAD or MI. Patients were eligible for inclusion in the post-MI cohort if they had been admitted to hospital with a diagnosis of MI that fulfilled the European Society of Cardiology (ESC) 4th Universal definition of type 1 MI. This is defined as acute myocardial injury with clinical evidence of acute myocardial ischaemia. A fall and/or rise of cardiac troponin (cTn) with 1 or more values beyond the 99th percentile of the Upper Reference Limit (URL), in addition to one or more of the following: evidence of new ischaemic ECG changes; pathological Q waves; current loss of myocardium or regional wall motion abnormality in line with an ischaemic aetiology; and coronary thrombus.

The study complied with ethical principles on human experimentation (Declaration of Helsinki) and was approved by the office for Research Ethics Committee Northern Ireland (ORECNI) (IRAS Reference-166742), and Research Governance Committee of Belfast Trust (Trust Reference-15144TM-AS). Written informed consent was obtained from each participant prior to recruitment. The study was conducted in accordance with Standard for Reporting Diagnostic Accuracy (STARD) guidelines.

*Clinical Data Collection.* All participants were asked to complete a clinical questionnaire detailing baseline demographics, past medical history, current medications, family history of medical conditions and lifestyle information. Electronic healthcare records were reviewed following ethical approval and participants' consent for medical history to ensure accuracy. Baseline measurement of blood pressure, heart rate, oxygen saturations, height, and weight were also performed. *Blood Sampling and Laboratory Methods.* For biomarker assessment, 24 ml of venous blood was sampled. Serum and plasma samples were analysed by Randox Clinical Laboratory Services (RCLS), (Antrim, UK) on cytokine arrays (Randox Laboratories Ltd, Crumlin, UK); using an Evidence Investigator analyser (Randox Laboratories Ltd, Crumlin, UK) for the following proteins: Cytokine array 1: Interleukin (IL)-1a, - 1β, -2, -4, -6, -8, -10, vascular endothelial growth factor (VEGF), epidermal growth factor (EGF), tumour necrosis factor alpha (TNFa), interferon gamma (IFNL) and monocyte chemoattractant protein-1 (MCP-1). H-FABP, adiponectin, homocysteine and HDL-3 were measured on the RX Imola analyser (Randox, Crumlin, UK). Folate and vitamin B₁₂ were measured on a Cobas8000 (Roche, Basil, Switzerland). Asymmetric dimethylarginine (ADMA) and leucine-rich alpha-2-glycoprotein 1 (LRG- 1) were assessed by ELISA, according to manufacturer's instructions (ELISAgenie, Ireland). The limit of detection (LOD) for the biomarkers were as follows: IL-1α 0.19 pg/ml; IL-1β 0.26 pg/ml; IL-2 2.97 pg/ml; IL-4 2.12 pg/ml; IL- 6 0.12 pg/ml; IL-8 0.36 pg/ml; IL-10 0.37 pg/ml, VEGF 3.24 pg/ml; EGF 1.04 pg/ml; TNF-α 0.59 pg/ml; IFN∟ 0.44 pg/ml; MCP-1 3.53 pg/ml; H-FABP <2.94 ng/ml; adiponectin 0.18 µg/ml; folate 2.72 nmol/l; homocysteine 1.74 µmol/l; , vitamin B₁₂ 73.8 pmol/l; HDL-3 4 mg/dl; plasma ADMA <0.938 µmol/L; plasma LRG-1 <0.0047 µg/ml. Results below the LOD were inputted as 90% of the LOD. Troponin, lipids, NT-proBNP, urate, urea and electrolytes and all other biochemical measurements, were analysed by clinical staff at the Kelvin laboratory, Royal Victoria Hospital, Belfast. Apo-lipoprotein A and B were analysed by Cardiff University, UK.

*Conjunctival Vessel Imaging.* Conjunctival microvascular imaging was performed using a non-invasive imaging tool: iPhone6s with slit lamp biomicroscope and semi-automated software developed by the VAMPIRE centre Dundee and NIBEC (Ulster University) for quantifying vessel haemodynamic properties (vessel diameter (D), axial velocity (Va), cross-sectional velocity (Vs), blood flow (Q), and wall shear rate (WSR)). Briefly, the imaging tool consisted of a 2X magnification device (Apple iPhone 6s) adapted as an eye piece of a slit-lamp biomicroscope (Topcon SL-D4) with a 40X magnification lens. A camera application, Pro Movie Recorder, was used for controlling camera settings such as focus, shutter speed and ISO. Videos were obtained from conjunctival vessels in the nasal and temporal bulbar conjunctivas of both eyes. Videos were captured at 60 frames per second and were analysed with set-up specific semi-automated software. Measurements from vessels of both eyes were combined for the estimation of overall mean of each parameter per patient (patient's mean).

*Estimation of Microcirculatory Parameters.* Estimation of blood vessel parameters was effected using video sequences with minimal motion artefact. The sharpest frame in the sequence was selected as a reference frame and all other frames registered to it. A segmentation algorithm was applied to segment vessels before vessel centrelines were extracted for estimating the dynamic properties of the vessels. Euclidean Distance Transformation was used for estimation of vessel diameter. The axial velocity (Va) was estimated based on spatial-temporal image (STI) via applying one dimension of space plus time (1D+T) continuous wavelet transform (1DTCWT). Blood flow was calculated from the product of the cross-sectional velocity (Vs) and diameter (D), using the formula, (Vs × πD2)/4. Wall shear rate was calculated by the formula 8 x Vs/D.

*Statistical Analysis.* Statistical analyses were performed using IBM SPSS v25. The following statistics were analysed on the appropriate data: descriptive statistics (mean ± sd) and percentages for summarizing parametric and nominal data, respectively; Chi square and Pearson's tests for determining the association and correlation between variables; and independent t-test for differences between group means for parametric data. Where the assumptions of t-test were unmet, the Mann-Whitney U test was used. Variable medians were compared when distributions were similar but mean ranks were compared when distributions were dissimilar. Binary logistic regression and receiver operating characteristic (ROC) curves were used to test sensitivity and specificity. Normality was assessed by Shapiro Wilks test (p>0.05). Data transformation was applied when the assumption of normality was unmet. Statistical significance was set at an α-level p<0.05.

### Results

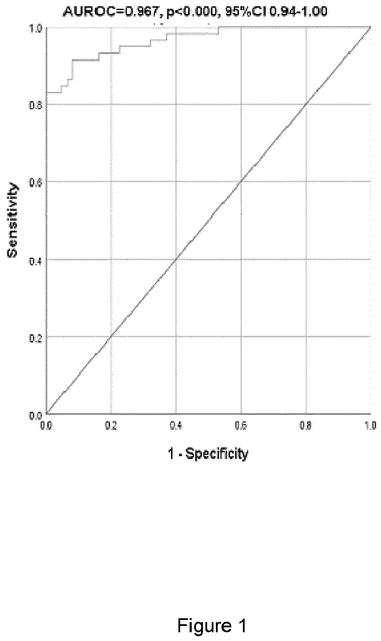

Combining biomarker and ocular measurements provides a method of identifying individuals at risk of CAD and MI. Using forward and backward Wald logistic regression the combination of Vs with NT-proBNP and adiponectin gave the highest predictive ability to discriminate the post-MI group from control, with the fewest variables suitable for the patient cohort size for the study (X2(3)=111.74, p<0.05). The model explained 80.4% (Nagelkerke R2) of the variance in CAD and correctly classified 90.1% of cases. Hosmer and Lemeshow test for goodness of fit was X2(8)=6.83, p=0.55. Sensitivity 93.0%, specificity 91.5%, PPV 91.4%, NPV 93.1%; ROC 0.967 (Table 4 and Figure 1). Furthermore, substitution of Va for Vs gave a similar level of diagnostic power. The use of a mobile device with photographic means, such as a mobile phone, to obtain the CMP data enables the method to be readily available for use at any location.

**Table 1: Demographic and Clinical Characteristics (mean ± SD)**

| **Variables** | **Control n=66** | **Post-MI n=66** | **p value** |
|---|---|---|---|
| Age (years) | 52.50±9.73 | 56.73±11.41 | 0.039 |
| Gender(males) | 45/66 (68.2%) | 52/66 (78.8%) | 0.168 |
| Height (cm) | 168.21±10.68 | 170.77±9.34 | 0.262 |
| Weight (kg) | 81.40±22.72 | 83.59±14.93 | 0.186 |
| Body mass index (kg/m²) | 28.71±7.66 | 28.62±4.68 | 0.306 |
| Systolic BP (mmHq) | 128±16 | 120±16 | 0.006 |
| Diastolic BP (mmHg) | 76±10 | 73±11 | 0.066 |
| Heart rate (beats/minute) | 71±10 | 73±12 | 0.299 |
| Hypertension | 8/66 (12.1%) | 31/66 (47.0%) | 0.001 |
| Diabetes | 2/66 (3.0%) | 15/66 (22.7%) | 0.001 |
| COPD | 6/66(9.1%) | 8/66 (12.1%) | 0.572 |
| Hypercholesterolemia | 17/64 (26.6%) | 36/66 (54.5%) | 0.001 |
| Previous MI | 0/66 (0%) | 9/66 (13.6%) | 0.002 |
| Heart failure | 0/66 (0%) | 6/65 (9.2%) | 0.012 |
| Stroke | 0/66 (0%) | 1/66 (1.5%) | 0.315 |
| Smoking (Yes) | 29/66 (43.9%) | 43/66 (65.2%) | 0.014 |
| Family history of IHD | 21/66 (31.8%) | 38/66 (57.6%) | 0.003 |

| | | | |
|---|---|---|---|
| COPD: chronic obstructive pulmonary disease, IHD: ischaemic heart disease | | | |

**Table 2: Conjunctival microvascular parameters (mean ±S D)**

| **Variables** | **Control n=66** | **Post-MI n=66** | **p value** |
|---|---|---|---|
| Diameter (D, µm) | 21.45±3.03 | 22.79±3.07 | 0.012 |
| Axial velocity (Va, mm/s) | 0.54±0.05 | 0.50±0.06 | 0.001 |
| Cross-sectional velocity (Vs, mm/s) | 0.38±0.04 | 0.35±0.04 | 0.001 |
| Blood flow (Q, pl/s) | 159.66±47.27 | 161±48.12 | 0.457 |
| Wall shear rate (WSR, s-1) | 167.78±34.37 | 143.59±28.56 | 0.001 |

**Table 3: Biomarker results (mean ± SD)**

| **Variables** | **Control n=66** | **Post MI n=66** | **p value** |
|---|---|---|---|
| HbA1c (mmol/l) | 39.25±8.29 | 45.27±16.89 | 0.053 |
| Sodium (mmol/l) | 140.28±1.77 | 138.95±2.52 | 0.001 |
| Potassium (mmol/l) | 4.40±0.31 | 4.33±0.36 | 0.401 |
| Urea (mmol/l) | 5.38±1.18 | 5.47±2.18 | 0.541 |
| Creatinine (µmol/l) | 79.28±16.66 | 84.05±22.21 | 0.272 |
| Creatinine clearance (ml/min) | 110±42.42 | 104.80±34.93 | 0.733 |
| HGB (g/l) | 146.38±11.00 | 142.39±15.44 | 0.095 |
| Haematocrit (l/l) | 0.43±0.03 | 0.42±0.04 | 0.145 |
| White cell count (10^{x9}µl/l) | 6.76±1.73 | 8.90±3.01 | 0.001 |
| Platelet count (10^{x9}/l) | 263.17±46.54 | 262.59±68.75 | 0.426 |
| MCV (fl) | 88.93±4.30 | 88.20±5.93 | 0.438 |
| CRP (mg/l) | 3.24±6.39 | 20.96±42.49 | 0.001 |
| NT-proBNP (ng/l) | 74.70±186.68 | 1049.11±1663.65 | 0.001 |
| Total Cholesterol (mmol/l) | 5.11±0.89 | 4.65±1.44 | 0.005 |
| Triglyceride (mmol/l) | 1.67±0.96 | 1.97±1.35 | 0.217 |
| HDL (mmol/l) | 1.48±0.48 | 1.13±0.30 | 0.001 |
| LDL (mmol/l) | 2.85±0.72 | 2.69±1.33 | 0.124 |
| Non-HDL (mmol/l) | 3.62±0.97 | 3.52±1.41 | 0.404 |
| Chol-HDL ratio | 3.79±1.37 | 4.31±1.58 | 0.048 |
| Prothrombin Time (secs) | 10.52±0.50 | 10.94±0.70 | 0.001 |
| APTT (secs) | 26.47±2.19 | 35.17±29.28 | 0.468 |
| Fibrinogen g/l) | 3.12±0.61 | 3.89±1.05 | 0.001 |
| Urate (mmol/l) | 0.32±0.07 | 0.35±0.11 | 0.280 |
| Apolipoprotein A (g/l) | 1.54±0.29 | 1.34±0.25 | 0.001 |
| Apolipoprotein B (g/l) | 1.02±0.23 | 1.04±0.31 | 0.949 |
| Folate (nmol/l) | 8.23±6.06 | 6.45±4.15 | 0.088 |
| Homocysteine (µmol/l) | 14.35±6.96 | 15.81±7.15 | 0.084 |
| Vitamin B₁₂ (pmol/l) | 436.97±210.39 | 396.92±174.68 | 0.348 |
| Adiponectin (µg/ml | 11.15±6.20 | 8.58±5.92 | 0.001 |
| H-FABP (ng/ml) | 3.62±2.04 | 13.75±29.73 | 0.001 |
| HDL-3 (mg/dl) | 20.17±4.51 | 17.73±4.75 | 0.001 |
| Serum IL-1α (pg/ml) | 0.48±0.13 | 0.47±0.13 | 0.320 |
| Serum IL-1β (pg/ml) | 2.38±2.77 | 1.98±0.50 | 0.550 |
| Serum IL-2 (pg/ml) | 4.25±5.62 | 3.46±1.37 | 0.673 |
| Serum IL-4 (pg/ml) | 2.80±1.77 | 2.43±0.47 | 0.332 |
| Serum IL-6 (pg/ml) | 1.92±2.36 | 8.17±16.77 | 0.001 |
| Serum IL-8 (pg/ml) | 10.87±8.40 | 13.37±13.56 | 0.082 |
| Serum IL-10 (pg/ml) | 1.38±0.71 | 1.39±0.46 | 0.469 |
| Serum MCP-1 (pg/ml) | 236.54±100.32 | 201.79±124.48 | 0.006 |
| Serum TNF-α (pg/ml) | 2.96±2.17 | 2.75±0.87 | 0.594 |
| Serum VEGF (pg/ml) | 120.62±102.64 | 162.15±123.21 | 0.043 |
| Serum IFN□ (pg/ml) | 0.69±0.67 | 0.98±2.91 | 0.523 |
| Serum EGF (pg/ml) | 80.79±41.54 | 60.89±38.96 | 0.011 |
| Plasma IL-1a (pg/ml) | 0.44±0.14 | 0.43±0.13 | 0.796 |
| Plasma 1L-1β (pg/ml) | 2.42±4.35 | 1.83±0.52 | 0.366 |
| Plasma IL-2 (pg/ml) | 4.03±5.23 | 3.31±1.08 | 0.982 |
| Plasma IL-4 (pg/ml) | 2.82±1.81 | 2.30±0.49 | 0.031 |
| Plasma IL-6 (pg/ml) | 1.99±3.00 | 7.06±10.88 | 0.001 |
| Plasma IL-8 (pg/ml) | 4.74±4.04 | 5.20±8.14 | 0.564 |
| Plasma IL-10 (pg/ml) | 1.29±0.46 | 1.43±0.52 | 0.094 |
| Plasma MCP-1 (pg/ml) | 90.41±37.16 | 87.13±70.29 | 0.103 |
| Plasma TNF-α (pg/ml) | 2.95±5.48 | 2.33±0.87 | 0.347 |
| Plasma VEGF (pg/ml) | 24.92±21.48 | 20.22±9.97 | 0.468 |
| Plasma IFN□ (pg/ml) | 0.63±0.57 | 1.04±2.88 | 0.478 |
| Plasma EGF (pg/ml) | 21.09±16.39 | 16.04±15.29 | 0.060 |
| ADMA (µmol/l) | 3.15±1.68 | 3.63±11.93 | 0.258 |
| LRG-1 (µg/ml) | 14.79±9.44 | 11.93±4.92 | 0.048 |

| | | | |
|---|---|---|---|
| HbA1c glycated haemoglobin; HGB: haemoglobin; MCV: corpuscular volume; HDL: highdensity lipoprotein, LDL: low-density lipoprotein; Cho-HDL: cholesterol-high-density lipoprotein; NT-proBNP: N-terminal pro brain natriuretic peptide; H-FABP: heart-type fatty acid-binding protein; CRP: C-reactive protein; IL: interleukin; MCP-1: monocyte chemoattractant protein-1; VEGF: vascular endothelial growth factor; TNF: tumour necrosis factor; IFN□: interferon gamma; EGF: endothelial growth factor, ADMA: asymmetrical dimethylarginine; LRG-1: leucine-rich alpha-2-glycoprotein- | | | |

**Table 4: Multivariate logistic regression with biomarkers and conjunctival parameters for detecting CAD**

| **Biomarkers/Ocular parameters** | **ROC** | **Sensitivity %** | **Specificity %** | **PPV %** | **NPV %** |
|---|---|---|---|---|---|
| log₁₀ NT-proBNP, log₁₀ adiponectin, Vs | 0.967 | 93.0 | 91.5 | 91.4 | 93.1 |
| log₁₀ NT-proBNP, log₁₀ adiponectin, Va | 0.966 | 94.7 | 93.2 | 93.1 | 94.8 |
| log₁₀ H-FABP, log₁₀ NT-proBNP, Va | 0.953 | 88.1 | 91.9 | 91.2 | 89.1 |
| log₁₀ H-FABP, log₁₀ NT-proBNP, Vs | 0.953 | 86.4 | 90.3 | 89.5 | 87.5 |
| log₁₀ H-FABP, log₁₀ NT-proBNP, log₁₀ WSR | 0.936 | 84.7 | 88.7 | 87.7 | 85.9 |
| log₁₀ NT-proBNP, Vs | 0.926 | 85.0 | 83.9 | 83.6 | 85.2 |
| log₁₀ NT-proBNP, Va | 0.925 | 83.3 | 85.5 | 84.7 | 84.1 |
| IL-6, Vs | 0.840 | 76.2 | 76.2 | 76.2 | 76.2 |
| log₁₀ H-FABP, Vs | 0.830 | 73.0 | 76.6 | 75.4 | 74.2 |
| log₁₀ H-FABP, Va | 0.815 | 73.0 | 71.9 | 71.9 | 73.0 |
| CRP, D | 0.810 | 79.4 | 67.2 | 71.4 | 75.9 |
| log₁₀ adiponectin, log₁₀ WSR | 0.769 | 76.3 | 60.3 | 66.2 | 71.4 |
| log₁₀ adiponectin, Va | 0.721 | 76.2 | 53.1 | 61.5 | 69.4 |
| log₁₀ adiponectin, D | 0.702 | 72.1 | 54.2 | 62.0 | 65.3 |

| | | | | | |
|---|---|---|---|---|---|
| CRP C-reactive protein, H-FABP heart-type fatty acid- binding protein, IL-6 interleukin-6, NTproBNT N-terminal pro-brain natriuretic peptide, D vessel diameter, Va axial velocity, Vs cross-sectional velocity, WSR wall shear rate, ROC receiver operator characteristic, PPV positive predictive value, NPV negative predictive value. | | | | | |

## Claims

1. A method of aiding cardiovascular disease diagnosis in an individual comprising determining the concentration of NT-proBNP and adiponectin in an *in vitro* biological sample obtained from the individual and in conjunction with measurement values relating to conjunctival haemodynamic parameters of the individual establishing the significance of the measured values in relation to cardiovascular disease.

2. The method of claim 1 in which the cardiovascular disease is either coronary artery disease or myocardial infarction.

3. The method of any of the preceding claims in which the conjunctival haemodynamic parameter is the conjunctival haemodynamic cross-sectional velocity or the conjunctival haemodynamic axial velocity data.

4. The method of claim 3 in which the level of NT-proBNP and adiponectin is greater in the *in vitro* biological sample of the patient than that of control values and the axial or cross-sectional conjunctival haemodynamic velocity measurement data of the patient is greater than that of a control value.

5. The method of any of claims 2 to 4 in which the NT-proBNP, adiponectin and conjunctival haemodynamic velocity measurement data is placed into a computer program incorporating an algorithm whose output indicates the presence or absence of coronary artery disease or myocardial infarction in the patient.

6. The method of any of the preceding claims wherein the *in vitro* biological sample is a blood, serum or plasma sample.

7. The method of any of the preceding claims in which the conjunctival haemodynamic parameter measurements are derived from a system that comprises a mobile device incorporating photographic means and a computer program for processing data captured by the mobile device.

8. The method of claim 7 in which the mobile device further comprises NT-proBNP and adiponectin measurement data derived from the *in vitro* biological sample.

9. The method of claims 7 and 8 in which the mobile device is a mobile phone.

10. A mobile device with photographic means which comprises a computer program incorporating an algorithm for processing data relating to haemodynamic conjunctival parameter, adiponectin and NT-proBNP measurements.

11. The mobile device of claim 10 in which the data for the haemodynamic conjunctival parameter is cross-sectional conjunctival haemodynamic velocity and/or axial conjunctival haemodynamic velocity.

12. The mobile device of any of claims 10 and 11 in which the data relating to adiponectin and NT-proBNP is their individual concentrations measured in an *in vitro* biological sample, preferably blood.

13. Use of the mobile device of any of claims 10 to 12 for supporting the diagnosis of cardiovascular disease and myocardial infarction.

14. A system for aiding the diagnosis myocardial infarction or coronary artery disease comprising a mobile device with photographic means which comprises a computer program for processing data relating to an haemodynamic conjunctival parameter measurement and communication means to a device incorporating a computer program incorporating an algorithm for processing data relating to a haemodynamic conjunctival parameter, adiponectin and NT-proBNP.
